# EUROPEAN PATENT SPECIFICATION

(11) **EP 2 757 159 B1**
(45) Date of publication and mention of the grant of the patent: **28.06.2017**
(21) Application number: 14151579.1
(22) Date of filing: 17.01.2014
(51) Int. Cl.: C12Q 1/06, C12M 1/34, C12Q 1/22

(54) **Microorganism detecting system and microorganism detecting method**
Mikroorganismuserkennungssystem und Verfahren zur Erkennung
Système de détection de micro-organismes et procédé de détection de micro-organismes

(30) Priority: 17.01.2013 JP 2013006622
(43) Date of publication of application: 23.07.2014
(73) Proprietor: Azbil Corporation, Chiyoda-ku Tokyo 100-6419 (JP)
(72) Inventor: Yamasaki, Shinsuke, Tokyo, Tokyo 100-6419 (JP); Obara, Daisuke, Tokyo, Tokyo 100-6419 (JP); Furuya, Masashi, Tokyo, Tokyo 100-6419 (JP)
(74) Representative: Lavoix

(56) References cited:
- WO-A2-01/36661
- JP-A- H06 181 743
- US-A- 5 739 003
- BANADA P P ET AL: "Label-free detection of multiple bacterial pathogens using light-scattering sensor", BIOSENSORS AND BIOELECTRONICS, ELSEVIER BV, NL, vol. 24, no. 6, 15 February 2009 (2009-02-15), pages 1685-1692, XP025922743, ISSN: 0956-5663, DOI: 10.1016/J.BIOS.2008.08.053 [retrieved on 2008-09-11]

## Description

The present invention relates to a quality evaluating technology, and, in particular, relates to a microorganism detecting system and microorganism detecting method.

In purified water, water for pharmaceutical manufacturing, water for injection, and the like, treatment reference values before microorganisms have been established in the Pharmacopeia. These solutions are manufactured through, for example, distillation, ultrafiltration, the use of a reverse osmosis membrane, or the like (referencing, for example, Japanese Patent 2997099, WO 2008/038575 A, JP 2012 -192315). During manufacturing, quality of these solutions is controlled through monitoring electric conductivity and TOC (Total Organic Carbon). For example, when manufacturing water for an injection through distillation, care is taken so as to not produce contamination through entrainment. Moreover, after these solutions are manufactured, a portion of the solution is extracted and inoculated into a culture medium to examine the number of colonies of the microorganisms that are produced under specific conditions. Whether or not microorganisms in the solution that has been manufactured are below the treatment standard values is inspected thereby.

However, the electrical conductivity and TOC of the solution do not necessarily reflect quantitatively the number of microorganisms included in the solution. Moreover, when it is discovered, after the solution is manufactured, that the number of microorganism colonies that are formed is too large, it is necessary to dispose of the solution that has been manufactured, resulting in the waste of the manufacturing cost and time. Given this, one object of the present invention is to provide a microorganism detecting system and microorganism detecting method that are able to estimate, in a short time, the number of colonies that may be formed by the microorganisms included in the solution. JP H 06-181743 relates an apparatus for the determination of the number of live microbial cells.

The present invention provides a microorganism detecting system according to claim 1.

The present invention provides a microorganism detecting method according to claim 8.

The present invention enables the provision of a microorganism detecting system and a microorganism detecting method that is able to estimate, in a short time, the number of microorganism colonies that may be formed.

### Brief Descriptions of the Drawings

So that the manner in which the above recited features of the present invention can be understood in detail, a more particular description of the invention, briefly summarized above, may be read by reference to embodiments. The accompanying drawings relate to embodiments of the invention and are described in the following:

FIG. 1 is a schematic diagram of a microorganism detecting system according to an embodiment according to the present invention.
FIG. 2 is a graph illustrating the fluorescent intensity for each of various types of microorganisms in an embodiment according to the present invention.
FIG. 3 is a graph illustrating schematically the relationship between the fluorescent intensity and the particle diameters of microorganisms within a solution, in an embodiment according to the present invention.
FIG. 4 is a table showing examples of the correlation relationships between the number of microorganisms in a solution and the number of colonies that the microorganisms will form in a culture medium in an embodiment according to the present invention.
FIG. 5 is a schematic diagram of an optical microorganism detecting device according to an embodiment according to the present invention.
FIG. 6 is a graph illustrating the correlation relationships between the numbers of microorganisms in a solution and the numbers of colonies that the microorganisms will form on a culture medium.

A form of embodiment of the present invention will be described below. In the descriptions of the drawings below, identical or similar components are indicated by identical or similar codes. Note that the diagrams are schematic. Consequently, specific measurements should be evaluated in light of the descriptions below. Furthermore, even within these drawings there may, of course, be portions having differing dimensional relationships and proportions.

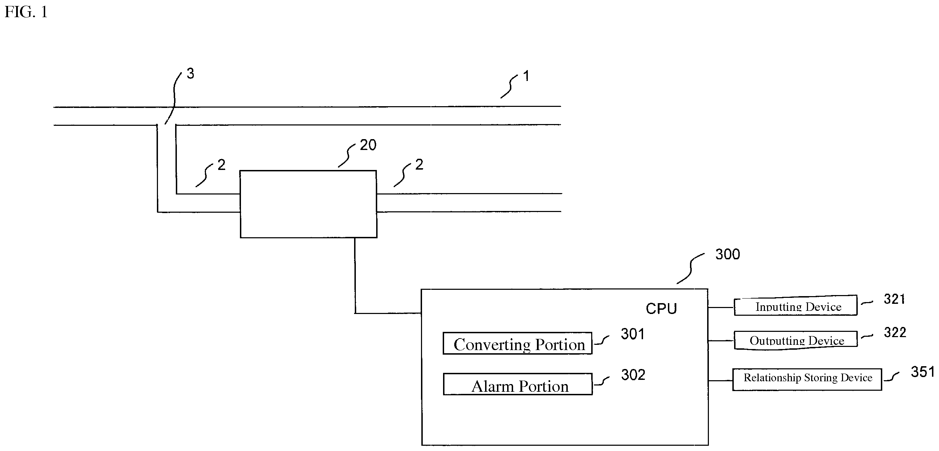

A microorganism detecting system according to an embodiment, as illustrated in FIG. 1, comprises: a microorganism detecting device 20 for detecting the number of microorganisms that are included within the solution, through illuminating the solution with light and detecting the florescent light that is emitted by microorganisms that are included in the solution; a relationship storing device 351 for storing a correlation relationship between the number of microorganisms within a solution and the number of colonies that are formed by the microorganisms on a culture medium; and a converting portion 301 for converting, into a number of colonies that may be formed, the number of microorganisms detected by the microorganism detecting device 20, using the correlation relationship. The converting portion 301 includes a central calculation processing device (CPU) 300. The relationship storing device 351 is connected to the CPU 300.

The solution flows through a main pipe 1. A branch pipe 2 is connected to the main pipe 1 through a sampling pipe 3. A portion of the solution that flows through the main pipe 1 flows into the branch pipe 2. The solution is, for example, distilled water that is being manufactured or that has been manufactured, water for pharmaceutical manufacturing, water for injection, and the like, although there is no limitation thereto.

The microorganism detecting device 20 is provided in the branch pipe 2. As a result, a portion of the solution is introduced into the microorganism detecting device 20 from the main pipe 1 wherein the solution is flowing. While, as the microorganism detecting device 20, a device that is disclosed in US Patent 7430046 may be used, there is no limitation thereto. For example, the microorganism detecting device 20 may be provided with a light source, such as a laser. The light source emits a light, such as a laser beam, toward the solution. The light may be visible light or may be ultraviolet light. In the case of the light being visible light, the wavelength of the light is in the range of for example, between 400 and 550 nm, for example, 405 nm. In the case of the light being ultraviolet light, the wavelength of the light is in the range of, for example, between 310 and 380 nm, for example, 340 nm. Moreover, the microorganism detecting device 20 is provided with an optics system for focusing the emitted light onto a focal point, a pipe for carrying the fluid to the focal point, and the like.

If here microorganisms, including bacteria, are included in the solution, then the nicotinamide adenine dinucleotides and riboflavin, and the like, that are included in the microorganisms that are illuminated by the light will emit florescent light. Moreover, when the solution includes particles that include microbes and abiotic substances, the light that strikes the particles will be scattered through Mie scattering, producing scattered light.

Examples of such microbes include Gram-negative bacteria, Gram-positive bacteria, and fungi such as mold spores. *Escherichia coli*, for example, can be listed as an example of a Gram-negative bacterium. *Staphylococcus epidermidis*, *Bacillus atrophaeus*, *Micrococcus lylae*, and *Corynebacterium afermentans* can be listed as examples of Gram-positive bacteria. *Aspergillus niger* can be listed as an example of a fungus such as a mold spore. However, the microorganisms that are detected by the microorganism detecting device 20 are not limited thereto.

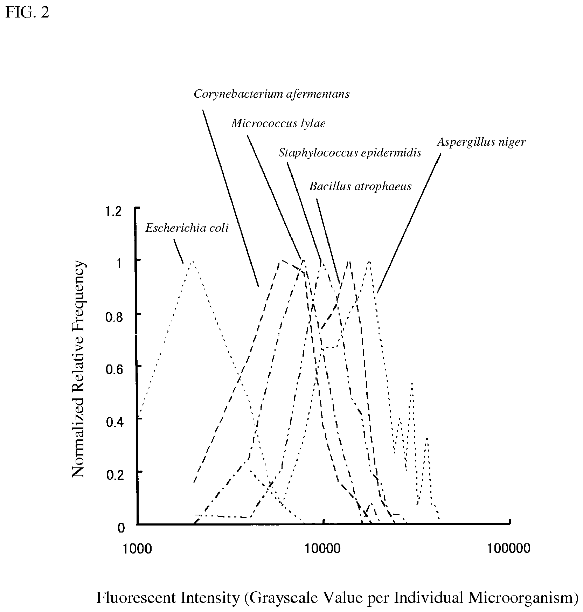

The microorganism detecting device 20 is further provided with a florescent light detecting device and a scattered light detecting device. The fluorescent light detecting device detects florescent light that is produced by the microorganisms, and measures the fluorescent intensity. The fluorescent light detecting device is able to measure the number of microorganisms included in the solution from the number of times that florescent light is detected. Moreover, as illustrated in FIG. 2, the intensity of the fluorescent light that is produced by the microorganism will differ depending on the type of microorganism. Because of this, it is possible to identify the type of microorganism that is included in the solution from the intensity of the fluorescent light that is detected by the florescent light detecting device of the microorganism detecting device 20 that is illustrated in FIG. 1.

The scattered light detecting device detects the light that is scattered by the particles that are included in the solution. The scattered light detecting device is able to count the number of particles from the number of times that scattered light is detected. Moreover, the intensity of the light that is scattered from the particles is correlated to the diameters of the particles. Consequently, it is possible to calculate the diameter of a particle that is included in the solution by detecting the intensity of the scattered light that is detected by the scattered light detecting device. Moreover, the particle diameters of microorganisms are different depending on the type of microorganism. Because of this, it is possible to identify the type of microorganism that is included in the solution from the intensity of the scattered light that is detected by the scattered light detecting device.

When scattered light is detected by the scattered light detecting device and florescent light is not detected by the florescent light detecting device, it is understood that the particle that is included in the solution is a particle of an abiotic substance. If scattered light is detected by the scattered light detecting device and florescent light is detected by the florescent light detecting device, it is understood that a microorganism is included in the solution. Here particles of abiotic substances include, for example, non-toxic or toxic chemical substances, and various types of dust, and the like.

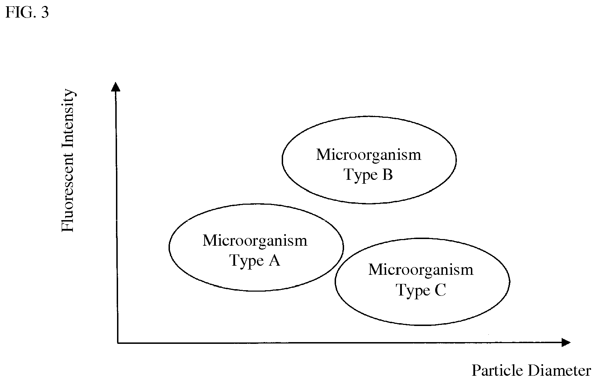

The microorganism detecting device 20 identifies the type of microorganism included in the solution using the detected fluorescent intensity and/or the particle diameter that is based on the reflected light intensity. Methods for identifying the type of microorganism based on both the fluorescent intensity and the scattered light intensity are disclosed in US Patent 6885440 and US patent 7106442, although there is no limitation thereto. For example, as illustrated in FIG. 3, correlations can be seen with the particle diameters and fluorescent intensities depending on the type of microorganism. Consequently, it is possible to identify the type of microorganism from the fluorescent intensity and the particle diameter through obtaining in advance a graph such as illustrated in FIG. 3.

Moreover, the microorganism detecting device 20 illustrated in FIG. 1 uses the number of times that florescent light is detected and/or the number of times that scattered light is detected, to identify the number of microorganisms included in a specific volume of the solution. For example, it is possible to identify the number of microorganisms included in a specific volume of the solution, for the solution that flows in the main pipe 1, from the flow rate ratio of the flow rate of the solution that flows in the main pipe 1 and the flow rate of the solution that flows in the branch pipe 2. The flow rates of the solutions that flow respectively in the main pipe 1 and the branch pipe 2 can be measured using a flow meter. Note that the microorganism detecting device 20 may instead detect the density of microorganisms in the solution as the number of microorganisms that are included in a specific volume of the solution.

The microorganism detecting device 20 sends, to the CPU 300, the types of microorganism detected and the number of microorganisms that are included in the specific volume of the solution. Note that in a case wherein the number of microorganisms can be identified but the types of microorganism could not be identified, the microorganism detecting device 20 sends, to the CPU 300, a signal indicating that the types of microorganism could not be identified, along with the number of microorganisms that are included in the specific volume of the solution.

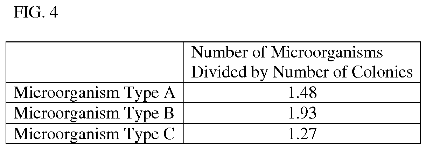

The relationship storing device 351 stores the ratio of the number of microorganisms in the solution to the number of colonies that may be formed by the microorganisms on a culture media, for each type of microorganism, such as is illustrated in FIG. 4, for example, as a correlation relationship between the number of microorganisms in the solution and the number of colonies that would be formed by the microorganisms on a culture medium. Note that the correlation relationship may be expressed as a high-order function. The units for the number of colonies that are formed by the microorganisms on the culture medium may be, for example, CFUs (Colony Forming Units). The correlation relationship between the number of microorganisms in the solution and the number of colonies that may be formed by the microorganisms on the culture medium may be obtained in advance through, for example, the procedure set forth below. First a plurality of solutions with different numbers of microorganisms included therein are prepared in advance. Following this, the numbers of microorganisms included in the respective solutions that have been prepared are detected by the microorganism detecting device. Moreover, the plurality of solutions that have yet prepared are inoculated onto respective culture media, and the microorganisms are cultured under specific culturing conditions, and the number of colonies formed are counted. Thereafter, a correlation relationship is calculated for the number of microorganisms that are detected by the microorganism detecting device and the number of colonies that are formed by the microorganisms on the culture medium. Here the microorganism detecting device, the sampling port, the volume of the branch pipe, the cleaning conditions for the main pipe, and the like, when obtaining the correlation relationship preferably are the same as when detecting the microorganisms by the microorganism detecting device 20, as illustrated in FIG. 1.

Note that the number of colonies that are formed by the microorganisms will differ depending on the culturing conditions. Because of this, when, for example, standard values for the number of colonies formed are established by law or by regulation, or the like, the culturing conditions when obtaining the correlation relationship must be in accordance with the culturing conditions specified by the law, regulation, or the like that establishes the standard values for the number of colonies formed. The culturing conditions include whether or not the solution that is inoculated onto the culture media is diluted or condensed, whether or not the microorganisms are collected using a membrane filter, the method for storing the solution prior to inoculation onto the culture medium, the type of culture medium, the duration of culturing, the temperature for culturing, and the like.

The converting portion 301 that is included in the CPU 300 that is illustrated in FIG. 1 reads out, from a relationship storing device 351, the correlation relationship between the number of microorganisms in the solution and the number of colonies that may be formed by the microorganisms on the culture medium, depending on the type of microorganism identified by the microorganism detecting device 20. For example, when the type of microorganism detected by the microorganism detecting device 20 is the "Microorganism Type A" shown in FIG. 4 and the number of "Microorganism Type A" included in the specific volume of the solution is 34, then the converting portion 301 reads out a ratio of 1.48 for "Microorganism Type A" in the solution to the number of colonies formed by the "Microorganism Type A" on the medium. Given this, the converting portion 301 divides by 1.48 the 34 that is the number of "Microorganism Type A" that is included in the specific volume of the solution, to calculate the number of colonies that may be formed by the "Microorganism Type A" on the culture medium as 23.

Moreover, when the type of microorganism detected by the microorganism detecting device 20 is the "Microorganism Type B" shown in FIG. 4 and the number of "Microorganism Type B" included in the specific volume of the solution is 54, then the converting portion 301 reads out a ratio of 1.93 for "Microorganism Type B" in the solution to the number of colonies formed by the "Microorganism Type B" on the medium. Given this, the converting portion 301 divides by 1.93 the 54 that is the number of "Microorganism Type B" that is included in the specific volume of the solution, to calculate the number of colonies that may be formed by the "Microorganism Type B" on the culture medium as 28.

Conversely, when the type of microorganism detected by the microorganism detecting device 20 is the "Microorganism Type C" shown in FIG. 4 and the number of "Microorganism Type C" included in the specific volume of the solution is 33, then the converting portion 301 reads out a ratio of 1.27 for "Microorganism Type C" in the solution to the number of colonies formed by the "Microorganism Type C" on the medium. Given this, the converting portion 301 divides by 1.27 the 33 that is the number of "Microorganism Type C" that is included in the specific volume of the solution, to calculate the number of colonies that may be formed by the "Microorganism Type C" on the culture medium as 26.

Note that in the event that despite detecting the number of microorganisms included in the specific volume of the solution, the microorganism detecting device 20 is unable to identify the type of microorganism, the converting portion 301 reads out, from the relationship storing device 351, the correlation relationship wherein the number of colonies formed by the microorganisms on the culture medium will be the greatest when converting from the number of microorganisms in the solution. The ratio of the number of microorganisms in the solution to the number of colonies that may be formed by the microorganisms on the culture media is read out as 1.27 as the correlation relationship wherein the number of colonies formed by the microorganisms on the culture medium will be the greatest, when converting from the number of microorganisms in the solution in the example illustrated in FIG. 4. Thereafter, the ratio 1.27 is used to calculate the number of colonies that may be formed on the culture medium by the microorganism for which the type could not be identified. This makes it possible to avoid the danger of underestimating the number of colonies that may be formed.

Note that, depending on the objective, when the microorganism detecting device 20 is unable to identify the type of microorganism, the converting portion 301 may instead use the correlation relationship wherein the converted number of colonies is the smallest. Conversely, the converting portion 301 may instead calculate an average value for the number of colonies converted using all of the correlation relationships. Furthermore, the ratios of the number of microorganisms in the solution to the number of colonies that may be formed by the microorganisms on the culture media may be set as appropriate depending on the objective. Moreover, when the microorganism detecting device 20 has identified a plurality of types of microorganisms, the converting portion 301 may calculate the number of colonies that may be formed by each respective type of microorganism, and then may take a total thereof.

An inputting device 321 and an outputting device 322 are connected to the CPU 300. A keyboard, a mouse, and the like, can be used for the inputting device 321. The inputting device 321 is used, for example, when storing the correlation relationships in the relationship storing device 351. Moreover, a display, a speaker, and the like may be used for the outputting device 322. The outputting device 322 outputs the number of colonies that may be formed by the microorganisms on the culture medium, calculated by the converting portion 301.

The CPU 300 is further provided with an alarm portion 302. The alarm portion 302 issues an alarm, through, for example, the outputting device 322 that is connected to the CPU 300, when the microorganism detecting device 20 has detected a microorganism that produces a toxin, such as an endotoxin, or if the number of calculated colonies, calculated by the converting portion 301, exceeds the standard value.

The microorganism detecting system according to the present embodiment, described above, makes it possible to estimate instantly the number of colonies that may be formed when culturing the microorganisms that are included in the solution. This makes it possible to estimate in real time the number of colonies that may be formed when culturing the microorganisms that are included in the purified water, the water for pharmaceutical manufacturing, the water for injection, and the like, during manufacturing in a pharmaceutical plant. Moreover, this enables prompt action, such as cleaning of the equipment for manufacturing the solution, to be taken when the estimated number of colonies that may be formed is above the standard established by law or regulation.

Moreover, the microorganisms detected by the microorganism detecting device 20 may be cultured in an incubator. For example, at least a portion of the solution that is provided to the microorganism detecting device 20 may be inoculated onto a culture medium, or microbe collection may be performed using a membrane filter, and the microorganisms may be cultured to perform verification, visually or using a microscope, of the types of microorganisms and the number of colonies that are actually formed.

### Examples of Embodiments

While a more specific description will be provided below through an embodiment in an example of embodiment, the present invention is in no wise limited by the example of embodiment set forth below.

*Bacillus atrophaeus, Escherichia coli,* and *Staphylococcus epidermidis* were prepared as the microorganisms. Following this, a plurality of solutions including the prospective microbes was prepared. The microbe densities were varied in the respective solutions. Note that purified water was used as the solvent for the solutions. Following this, an optical microorganism detecting device was used to count the number of microorganisms included per 1 mL of the respective solutions.

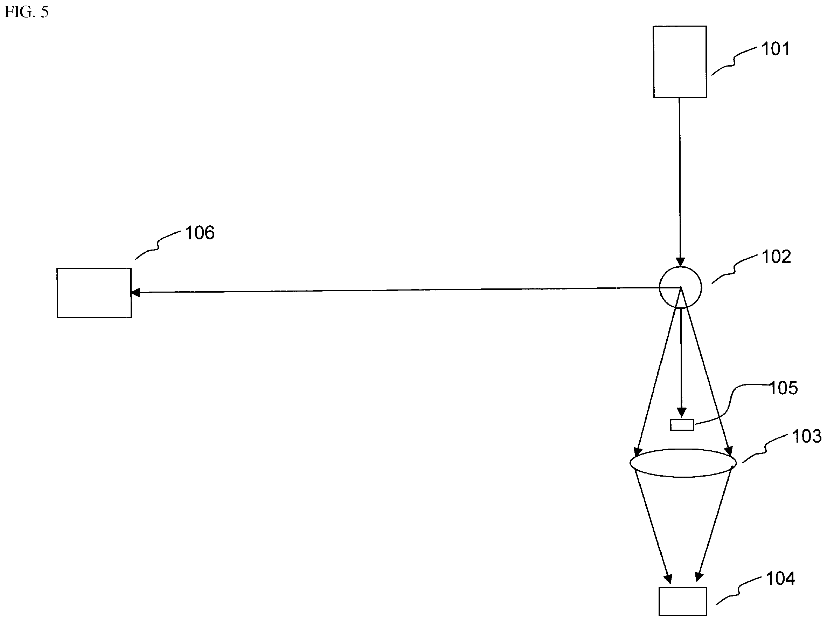

Note that, as illustrated in FIG. 5, the optical microorganism detecting device according to the present embodiment comprises: a light source 101, a pipe 102 that is illuminated by the light emitted from the light source, that is a pipe 102 wherein the solution that contains the microorganisms flows, and is a pipe 102 of a flow cell, or the like, made from a transparent material, such as glass; a lens 103 for focusing the scattered light that is produced by the microorganisms within the pipe 102; a scattered light detecting device 104 for detecting scattered light focused by the lens 103; and a blocking plate for blocking the scattered light detecting device 104 from light other than the scattered light; and a florescent light detecting device 106 for detecting the florescent light that is produced by the microorganisms in the pipe 102.

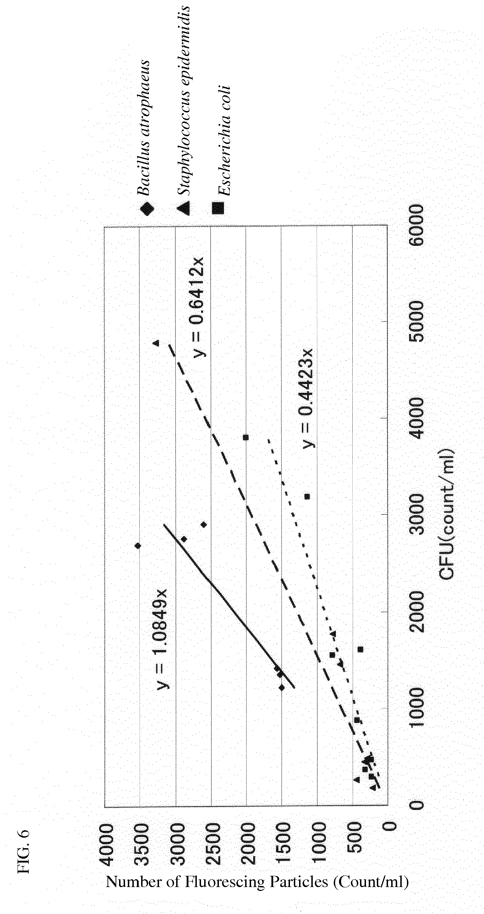

Following this, trypcase soy agars, manufactured by a Eiken Chemical Co., Ltd., were prepared as the culture media, and 80 mL each of the plurality of solutions were filtrated and cultivated on the culture media. Thereafter, the microbes were cultured for 24 hours under 32°C temperature conditions, and the number of colonies of microbes formed were counted visually. Thereafter, for each type of microbe, the numbers of microbes in the solution and the numbers of colonies formed were plotted, and the correlation relationships thereof were approximated through the least-squares method as linear relationships, at which time the numbers of microbes in the solution and the numbers of colonies formed had the proportional relationships shown in FIG. 6.

### List of reference signs

1: Main Pipe
2: Branch Pipe
3: Sampling Port
20: Microorganism Detecting Device
101: Light Source
102: Pipe
103: Lens
104: Scattered Light Detecting Device
105: Blocking Plate
106: Fluorescent Light Detecting Device
300: Central Calculation Processing Device
301: Converting Portion
302: Alarm Portion
321: Inputting Device
322: Outputting Device
351: Relationship Storing Device

## Claims

1. A microorganism detecting system comprising:
a microorganism detecting device (20) adapted to illuminate a solution with light, to detect fluorescent light that is produced by a microorganism included in the solution, and to detect a number of microorganisms included in the solution from the number of times that fluorescent light is detected;
a relationship storing device (351) that stores a correlation relationship between a number of microorganisms in a solution and a number of colonies formed by the microorganisms on a culture medium; and
a converting portion (301) that uses the correlation relationship to convert, into a number of colonies that may be formed, the number of microorganisms detected by the microorganism detecting device.

2. The microorganism detecting system as set forth in Claim 1, wherein:
the correlation relationship is a ratio of a number of microorganisms in a solution and a number of colonies formed by the microorganisms on a culture medium.

3. The microorganism detecting system as set forth in Claim 1 or Claim 2, wherein:
the microorganism detecting device (20) identifies the type of a microorganism based on fluorescent intensity of florescent light produced by the microorganism and/or based on scattered light scattered by the microorganism;
the relationship storing device (351) stores the correlation relationship for each individual type of microorganism; and
the converting portion (301) converts, to a number of colonies that may be formed, the number of the microorganisms detected by the microorganism detecting device, using the correlation relationship corresponding to the type of microorganism identified by the microorganism detecting device.

4. The microorganism detecting system as set forth in any one of Claim 1 through Claim 3, further comprising:
an incubator that cultures the microorganism detected by the microorganism detecting device (20).

5. The microorganism detecting system as set forth in any one of Claim 1 through Claim 4, further comprising:
a membrane filter that collects the microorganisms detected by the microorganism detecting device.

6. The microorganism detecting system as set forth in any one of Claim 1 through Claim 5, wherein:
the solution is water for pharmaceutical manufacturing,
the solution is water for an injection, or
the solution is purified water.

7. The microorganism detecting system as set forth in any one of Claim 1 through Claim 6, wherein:
a branch pipe (2) for directing a portion of the solution to the microorganism detecting device (20) from the main pipe in which the solution flows.

8. A microorganism detecting method comprising:
a illumination step comprising illuminating a solution with light, detecting fluorescent light that is produced by a microorganism included in the solution, and detecting a number of microorganisms included in the solution from the number of times that fluorescent light is detected;
a correlation relationship preparation step of preparing a correlation relationship between the number of the microorganisms in the solution and a number of colonies formed by the microorganisms on a culture medium are prepared; and
a converting step of converting the number of microorganisms detected into a number of colonies that may be formed, using the correlation relationship.

9. The microorganism detecting method as set forth in Claim 8, wherein:
the correlation relationship is a ratio of the number of the microorganisms in the solution and the number of the colonies formed by the microorganisms on the culture medium.

10. The microorganism detecting method as set forth in Claim 8 or Claim 9, wherein:
the type of the microorganism is identified based on fluorescent intensity of florescent light produced by the microorganism and/or based on scattered light produced by the microorganism; and
in the correlation relationship preparation step, the correlation relationship associates the type with a specific microorganism.

11. The microorganism detecting method as set forth in any one of Claim 8 through Claim 10, further including:
a cultivation step of cultivating a detected microorganism.

12. The microorganism detecting method as set forth in any one of Claim 8 through Claim 11, further including:
a collection step of collecting a detected microorganism by a membrane filter.

13. The microorganism detecting method as set forth in any one of Claim 8 through Claim 12, wherein:
the solution is water for pharmaceutical manufacturing,
the solution is water for an injection, or
the solution is purified water.

14. The microorganism detecting method as set forth in any one of Claim 8 through Claim 13, wherein:
the detection of the number of microorganisms is through a microorganism detecting device; and the microorganism detecting method further comprises the directing step of directing a portion of the solution to the microorganism detecting device from a main pipe in which the solution flows, using a branch pipe.

## Patentansprüche

1. Mikroorganismuserkennungssystem, das umfasst:
eine Mikroorganismuserkennungsvorrichtung (20), die so ausgelegt ist, dass sie eine Lösung mit Licht beleuchtet, um fluoreszierendes Licht zu erkennen, das von einem in der Lösung umfassten Mikroorganismus erzeugt wird, und um eine Anzahl von in der Lösung umfassten Mikroorganismen anhand der Anzahl von Malen, die fluoreszierendes Licht erkannt wird, zu erkennen;
eine Beziehungsspeichervorrichtung (351), die eine Korrelationsbeziehung zwischen einer Anzahl von Mikroorganismen in einer Lösung und einer Anzahl von Kolonien, die von den Mikroorganismen auf einem Kulturmedium gebildet werden, speichert; und
einen Umwandlungsabschnitt (301), der die Anzahl von Mikroorganismen, die von der Mikroorganismuserkennungsvorrichtung erkannt werden, unter Verwendung der Korrelationsbeziehung in eine Anzahl von Kolonien, die sich ggf. bilden, umwandelt.

2. Mikroorganismuserkennungssystem nach Anspruch 1, wobei:
die Korrelationsbeziehung ein Verhältnis einer Anzahl von Mikroorganismen in einer Lösung und einer Anzahl von Kolonien, die von den Mikroorganismen auf einem Kulturmedium gebildet werden, ist.

3. Mikroorganismuserkennungssystem nach Anspruch 1 oder 2, wobei:
die Mikroorganismuserkennungsvorrichtung (20) den Typ eines Mikroorganismus auf Basis einer Fluoreszenzintensität von fluoreszierendem Licht, das vom Mikroorganismus erzeugt wird, und/oder auf Basis von zerstreutem Licht, das vom Mikroorganismus zerstreut wird, identifiziert;
die Beziehungsspeichervorrichtung (351) die Korrelationsbeziehung für jeden einzelnen Typ von Mikroorganismus speichert; und
der Umwandlungsabschnitt (301) die Anzahl der Mikroorganismen, die von der Mikroorganismuserkennungsvorrichtung erkannt werden, unter Verwendung der Korrelationsbeziehung, die dem von der Mikroorganismuserkennungsvorrichtung identifizierten Typ von Mikroorganismus entspricht, in eine Anzahl von Kolonien, die ggf. gebildet werden, umwandelt.

4. Mikroorganismuserkennungssystem nach einem der Ansprüche 1 bis 3, das ferner umfasst:
einen Inkubator, der den von der Mikroorganismuserkennungsvorrichtung (20) erkannten Mikroorganismus kultiviert.

5. Mikroorganismuserkennungssystem nach einem der Ansprüche 1 bis 4, das ferner umfasst:
einen Membranfilter, der die von der Mikroorganismuserkennungsvorrichtung erkannten Mikroorganismen sammelt.

6. Mikroorganismuserkennungssystem nach einem der Ansprüche 1 bis 5, wobei:
die Lösung Wasser für pharmazeutische Zwecke ist,
die Lösung Wasser für Injektionszwecke ist oder
die Lösung gereinigtes Wasser ist.

7. Mikroorganismuserkennungssystem nach einem der Ansprüche 1 bis 6, wobei:
ein Abzweigrohr (2) zum Leiten eines Teils der Lösung zur Mikroorganismuserkennungsvorrichtung (20) vom Hauptrohr, in dem die Lösung fließt.

8. Mikroorganismuserkennungsverfahren, das umfasst:
einen Beleuchtungsschritt, der das Beleuchten einer Lösung mit Licht, das Erkennen von fluoreszierendem Licht, das von einem in der Lösung umfassten Mikroorganismus erzeugt wird, und das Erkennen einer Anzahl von in der Lösung umfassten Mikroorganismen anhand der Anzahl von Malen, die fluoreszierendes Licht erkannt wird, umfasst;
einen Korrelationsbeziehungsherstellungsschritt zum Herstellen einer Korrelationsbeziehung zwischen der Anzahl von Mikroorganismen in der Lösung und der Anzahl von Kolonien, die von den Mikroorganismen auf einem Kulturmedium gebildet werden, werden hergestellt; und
einen Umwandlungsschritt zum Umwandeln der Anzahl von erkannten Mikroorganismen in eine Anzahl von Kolonien, die ggf. gebildet werden, unter Verwendung der Korrelationsbeziehung.

9. Mikroorganismuserkennungsverfahren nach Anspruch 8, wobei:
die Korrelationsbeziehung ein Verhältnis der Anzahl der Mikroorganismen in der Lösung und der Anzahl der Kolonien, die von den Mikroorganismen auf dem Kulturmedium gebildet werden, ist.

10. Mikroorganismuserkennungsverfahren nach Anspruch 8 oder 9, wobei:
der Typ des Mikroorganismus auf Basis einer Fluoreszenzintensität von fluoreszierendem Licht, das vom Mikroorganismus erzeugt wird, und/oder auf Basis von zerstreutem Licht, das vom Mikroorganismus zerstreut wird, identifiziert wird; und
die Korrelationsbeziehung im Korrelationsbeziehungsherstellungsschritt den Typ mit einem spezifischen Mikroorganismus assoziiert.

11. Mikroorganismuserkennungsverfahren nach einem der Ansprüche 8 bis 10, das ferner umfasst:
einen Kultivierungsschritt zum Kultivieren eines erkannten Mikroorganismus.

12. Mikroorganismuserkennungsverfahren nach einem der Ansprüche 8 bis 11, das ferner umfasst:
einen Sammelschritt zum Sammeln eines erkannten Mikroorganismus mit einem Membranfilter.

13. Mikroorganismuserkennungsverfahren nach einem der Ansprüche 8 bis 12, wobei:
die Lösung Wasser für pharmazeutische Zwecke ist,
die Lösung Wasser für Injektionszwecke ist oder
die Lösung gereinigtes Wasser ist.

14. Mikroorganismuserkennungsverfahren nach einem der Ansprüche 8 bis 13, wobei:
das Erkennen der Anzahl von Mikroorganismen durch eine Mikroorganismuserkennungsvorrichtung erfolgt; und das Mikroorganismuserkennungsverfahren ferner den Leitschritt zum Leiten eines Teils der Lösung aus einem Hauptrohr, in dem die Lösung fließt, unter Verwendung eines Abzweigrohrs zur Mikroorganismuserkennungsvorrichtung umfasst.

## Revendications

1. Système de détection de micro-organismes comprenant :
un dispositif de détection de micro-organismes (20) adapté pour éclairer une solution à l'aide d'une lumière, pour détecter une lumière fluorescente qui est produite par un micro-organisme inclus dans la solution, et pour détecter un nombre de micro-organismes inclus dans la solution d'après le nombre de fois où la lumière fluorescente est détectée ;
un dispositif de stockage de relations (351) qui stocke une relation de corrélation entre un nombre de micro-organismes dans une solution et un nombre de colonies formées par les micro-organismes sur un milieu de culture ; et
une partie de conversion (301) qui utilise la relation de corrélation pour convertir, en un nombre de colonies susceptibles de se former, le nombre de micro-organismes détectés par le dispositif de détection de micro-organismes.

2. Système de détection de micro-organismes selon la revendication 1, dans lequel :
la relation de corrélation est un rapport entre un nombre de micro-organismes dans une solution et un nombre de colonies formées par les micro-organismes sur un milieu de culture.

3. Système de détection de micro-organismes selon la revendication 1 ou la revendication 2, dans lequel :
le dispositif de détection de micro-organismes (20) identifie le type d'un micro-organisme sur la base de l'intensité de la fluorescence de la lumière fluorescente produite par le micro-organisme et/ou sur la base de la lumière diffusée par le micro-organisme ;
le dispositif de stockage de relations (351) stocke la relation de corrélation pour chaque type individuel de micro-organisme ; et
la partie de conversion (301) convertit, en un nombre de colonies susceptibles de se former, le nombre des micro-organismes détectés par le dispositif de détection de micro-organismes, à l'aide de la relation de corrélation correspondant au type de micro-organisme identifié par le dispositif de détection de micro-organismes.

4. Système de détection de micro-organismes selon l'une quelconque de la revendication 1 à la revendication 3, comprenant en outre :
un incubateur qui sert à cultiver le micro-organisme détecté par le dispositif de détection de micro-organismes (20).

5. Système de détection de micro-organismes selon l'une quelconque de la revendication 1 à la revendication 4, comprenant en outre :
un filtre à membrane qui recueille les micro-organismes détectés par le dispositif de détection de micro-organismes.

6. Système de détection de micro-organismes selon l'une quelconque de la revendication 1 à la revendication 5, dans lequel :
la solution est de l'eau pour préparation pharmaceutique,
la solution est de l'eau pour injection, ou
la solution est de l'eau purifiée.

7. Système de détection de micro-organismes selon l'une quelconque de la revendication 1 à la revendication 6, dans lequel :
un tuyau de dérivation (2) sert à diriger une partie de la solution vers le dispositif de détection de micro-organismes (20) à partir du tuyau principal dans lequel la solution circule.

8. Procédé de détection de micro-organismes comprenant :
une étape d'éclairage comprenant l'éclairage d'une solution à l'aide d'une lumière, la détection de la lumière fluorescente qui est produite par un micro-organisme inclus dans la solution, et la détection d'un nombre de micro-organismes inclus dans la solution d'après le nombre de fois où la lumière fluorescente est détectée ;
une étape de préparation de relation de corrélation consistant à préparer une relation de corrélation entre le nombre des micro-organismes dans la solution et un nombre de colonies formées par les micro-organismes sur un milieu de culture ; et
une étape de conversion consistant à convertir le nombre de micro-organismes détectés en un nombre de colonies susceptibles de se former, à l'aide de la relation de corrélation.

9. Procédé de détection de micro-organismes selon la revendication 8, dans lequel :
la relation de corrélation est un rapport entre le nombre des micro-organismes dans la solution et le nombre des colonies formées par les micro-organismes sur le milieu de culture.

10. Procédé de détection de micro-organismes selon la revendication 8 ou la revendication 9, dans lequel :
le type du micro-organisme est identifié sur la base de l'intensité de la fluorescence de la lumière fluorescente produite par le micro-organisme et/ou sur la base de la lumière diffusée produite par le micro-organisme ; et
dans l'étape de préparation de relation de corrélation, la relation de corrélation associe le type à un micro-organisme particulier.

11. Procédé de détection de micro-organismes selon l'une quelconque de la revendication 8 à la revendication 10, incluant en outre :
une étape de culture consistant à cultiver un micro-organisme détecté.

12. Procédé de détection de micro-organismes selon l'une quelconque de la revendication 8 à la revendication 11, incluant en outre :
une étape de recueil consistant à recueillir un micro-organisme détecté par un filtre à membrane.

13. Procédé de détection de micro-organismes selon l'une quelconque de la revendication 8 à la revendication 12, dans lequel :
la solution est de l'eau pour préparation pharmaceutique,
la solution est de l'eau pour injection, ou
la solution est de l'eau purifiée.

14. Procédé de détection de micro-organismes selon l'une quelconque de la revendication 8 à la revendication 13, dans lequel :
la détection du nombre de micro-organismes est réalisée par un dispositif de détection de micro-organismes ; et le procédé de détection de micro-organismes comprend en outre l'étape de direction consistant à diriger une partie de la solution vers le dispositif de détection de micro-organismes à partir d'un tuyau principal dans lequel la solution circule, à l'aide d'un tuyau de dérivation.
